(19) Europäisches Patentamt
European Patent Office
Office européen des brevets

(11) Veröffentlichungsnummer: **0 023 884**
**B1**

(12) # EUROPÄISCHE PATENTSCHRIFT

(45) Veröffentlichungstag der Patentschrift: **20.07.83**

(51) Int. Cl.³: **C 07 C 127/22**, A 01 N 47/34

(21) Anmeldenummer: **80810219.8**

(22) Anmeldetag: **07.07.80**

(54) **N-Phenyl-N'-benzoylharnstoffe, Verfahren zu ihrer Herstellung, diese Verbindungen enthaltende Mittel und ihre Verwendung zur Bekämpfung von Schädlingen.**

(30) Priorität: **11.07.79 CH 6475/79**
**22.11.79 CH 10417/79**
**03.06.80 CH 4286/80**

(43) Veröffentlichungstag der Anmeldung: **11.02.81 Patentblatt 81/6**

(45) Bekanntmachung des Hinweises auf die Patenterteilung: **20.07.83 Patentblatt 83/29**

(84) Benannte Vertragsstaaten: **AT BE CH DE FR GB IT LI NL**

(56) Entgegenhaltungen:
**EP-A-0 003 099**
**DE-A-2 601 780**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**

**EP 0 023 884 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

N-Phenyl-N'-benzoylharnstoffe, Verfahren zu ihrer Herstellung, diese Verbindungen enthaltende Mittel und ihre Verwendung zur Bekämpfung von Schädlingen

Die vorliegende Erfindung betrifft neue halogensubstituierte N-(3-Trifluormethyl-4-halogenalkoxy-phenyl)-N'-benzoylharnstoffe, Verfahren zu ihrer Herstellung und ihre Verwendung in der Schädlingsbekämpfung.

Die erfindungsgemässen halogensubstituierten N-(3-Trifluormethyl-4-halogenalkoxy-phenyl)-N'-benzoylharnstoffe haben die Formel I

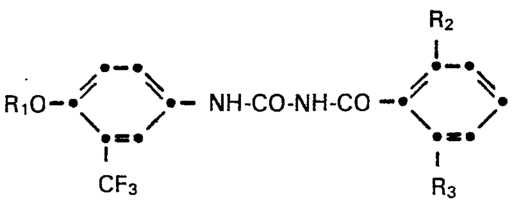

(I)

worin

$R_1$ einen mindestens zweifach mit Fluor, Chlor oder Brom substituierten $C_1$-$C_4$-Alkylrest;

$R_2$ Fluor oder Chlor; und

$R_3$ Wasserstoff, Fluor oder Chlor

bedeuten.

Wegen ihrer Wirkung als Schädlingsbekämpfungsmittel bevorzugt sind erfindungsgemässe Verbindungen der Formel I, die dadurch gekennzeichnet sind, dass $R_1$ die Gruppe $-CF_2-CHX_1X_2$ bedeutet, wobei $X_1$ für Fluor, Chlor oder Brom, vorzugsweise Fluor, und $X_2$ für Fluor, Chlor, Brom oder Trifluormethyl steht. Besondere Bedeutung haben Verbindungen der Formel I, worin $R_1$ für den Rest $-CF_2-CHF-CF_3$ steht.

Hervorzuheben sind auch Verbindungen der Formel I, worin $R_2$ und $R_3$ Fluor oder $R_2$ und $R_3$ Chlor bedeuten, sowie solche, worin $R_2$ Fluor oder Chlor und $R_3$ Wasserstoff bedeuten.

Die Verbindungen der Formel I können nach an sich bekannten Verfahren hergestellten werden (vgl. u.a. die deutschen Offenlegungsschriften Nummer 2 123 236, 2 601 780).

So kann man z.B. eine Verbindung der Formel I erhalten durch Umsetzung

a) einer Verbindung der Formel II

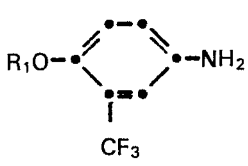

(II)

mit einer Verbindung der Formel III

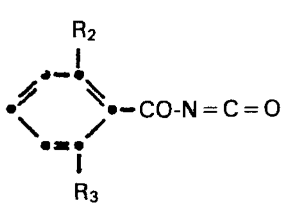

(III)

oder

b) einer Verbindung der Formel IV

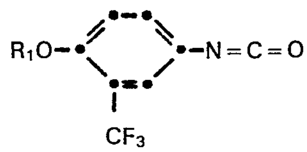

(IV)

gegebenenfalls in Gegenwart einer organischen oder anorganischen Base mit einer Verbindung der Formel V

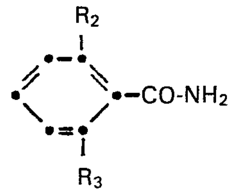

(V)

In den obigen Formeln II, III, IV und V haben die Reste $R_1$, $R_2$ und $R_3$ die unter Formel I vorstehend angegebenen Bedeutungen.

Die erwähnten Verfahren a) und b) können vorzugsweise unter normalem Druck und in Gegenwart eines inerten organischen Lösungs- oder Verdünnungsmittels durchgeführt werden. Als Lösungs- oder Verdünnungsmittel eignen sich z.B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Methyl-äthylketon, Methylisopropylketon und Methylisobutylketon. Verfahren a) wird im allgemeinen bei einer Temperatur von −10 bis 100°C, vorzugsweise zwischen 0 und 25°C, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, oder unter Zusatz eines Alkali- oder Erdalkalimetalls, vorzugsweise Natrium.

Die Ausgangsstoffe der Formeln II, III, IV und V sind bekannt oder können analog bekannten Verfahren hergestellt werden. So sind die substituierten Aniline der Formel II gemäss literaturbekannten Verfahren herstellbar, indem man z.B. 2-Trifluormethyl-4-nitrophenol [vgl. J. Org. Chem. *27* (1962), 4660] in Anwesenheit von Acetanhydrid hydriert und das entstandene 2-Trifluormethyl-4-acetaminophenol mit entsprechenden halogensubstituierten Alkenen analog der in Am. Soc. *73* (1951), 5831, beschriebenen Arbeitsweise veräthert. Anschliessend wird die N-Acetylgruppe in üblicher Weise abgespalten, um zu den Anilinen der Formel II zu gelangen. Einige dieser Aniline sind ferner in Analogie zu dem in J. Org. Chem. *29* (1964), 1, aufgezeigten Verfahren zugänglich (vgl. auch die dort zitierte Literatur).

Zu den Benzoylisocyanaten der Formel III kann

man unter anderem wie folgt gelangen (vgl. J. Agr. Food Chem. *21,* 348 und 993; 1973):

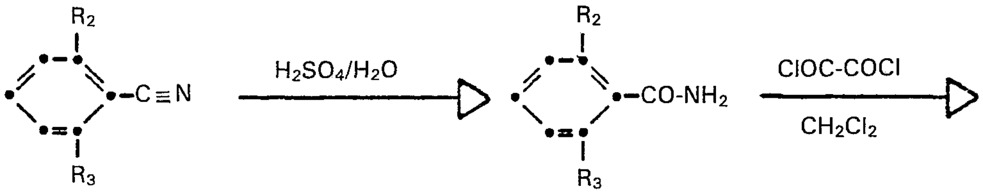


(III)

Die substituierten Phenylisocyanate der Formel IV lassen sich z.B. durch Phosgenisierung der entsprechenden Aniline der Formel II nach allgemein üblichen Verfahren herstellen. Die weiterhin als Ausgangsstoffe zu verwendenden Benzamide der Formel V sind zumeist bekannt (vgl. Beilstein «Handbuch der organischen Chemie» Bd. 9, S. 336).

Es ist bereits bekannt, dass bestimmte N-Phenyl-N'-benzoylharnstoffe insektizide Eigenschaften besitzen (vgl. deutsche Offenlegungsschrift Nr. 2 123 236, 2 504 982 und 2 537 413, die belgischen Patentschriften 832 304, 843 906 und 844 066 sowie die US-Patentschriften 4 085 226 und 4 089 975). Weiterhin werden in den deutschen Offenlegungsschriften 2 601 780, 2 726 684 und 2 820 696 insektizid wirksame halogensubstituierte N-Halogenalkoxyphenyl-N'-benzoylharnstoffe beschrieben.

Überraschenderweise wurde nun gefunden, dass die erfindungsgemässen N-(3-Trifluormethyl-4-halogenalkoxy-phenyl)-N'-benzoylharnstoffe der Formel I bei guter Pflanzenverträglichkeit und geringer Warmblütertoxizität höhere Wirksamkeit bei der Bekämpfung von Pflanzen und Tiere befallenden Schädlingen, vor allem gegen Insektenlarven, aufweisen als die vorerwähnten, aus dem Stand der Technik bekannten Verbindungen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Die Verbindungen der Formel I eignen sich vor allem zur Bekämpfung von pflanzenschädigenden Frassinsekten in Zier- und Nutzpflanzungen, insbesondere in Baumwollkulturen (z.B. Spodoptera littoralis und Heliothis virescens) sowie in Gemüsekulturen (z.B. Leptinotarsa decemlineata). Die Verbindungen der Formel I zeichnen sich durch eine ausgeprägte Wirkung gegen larvale Insektenstadien, insbesondere larvale Stadien fressender Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht: organische Phosphorverbindungen, Nitrophenole und Derivate, Formamidine. Harnstoffe, Carbamate, Pyrethroide und chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Träger und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind «cattle dips», d.h. Viehbäder, und «spray races», d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen:

Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate; Spritzpulver (wettable powders), Pasten, Emulsionen;

b) Lösungen.

Der Gehalt an Wirkstoff in den oben beschriebenen Mitteln liegt zwischen 0,1 bis 95%.

Die Wirkstoffe der Formel I können beispielsweise wie folgt formuliert werden:

*Stäubemittel:* Zur Herstellung eines a) 5%igen und b) 2%igen Stäubemittels werden die folgenden Stoffe verwendet:

a) 5 Teile Wirkstoff,
95 Teile Talkum,
b) 2 Teile Wirkstoff,
1 Teil hochdisperse Kiselsäure,
97 Teile Talkum.

Die Wirkstoffe werden mit den Trägerstoffen vermischt und vermahlen.

*Granulat:* Zur Herstellung eines 5%igen Granulates werden die folgenden Stoffe verwendet:

5,00 Teile Wirkstoff,
0,25 Teile epoxydiertes Pflanzenöl,
0,25 Teile Cetylpolyglykoläther,
3,50 Teile Polyäthylenglykol,
91,00 Teile Kaolin (Korngrösse 0,3-0,8 mm).

Die Aktivsubstanz wird mit dem epoxidierten Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

*Spritzpulver:* Zur Herstellung eines a) 40%igen, b) und c) 25%igen d) 10%iger Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;
b) 25,0 Teile Wirkstoff,
4,5 Teile Calcium-Ligninsulfonat,
1,9 Teile Champagne-Kreide/Hydroxyäthylcellulose-Gemische (1:1),
1,5 Teile Natrium-dibutyl-naphthalinsulfonat,
19,5 Teile Kieselsäure,
19,5 Teile Champagne-Kreide,
28,1 Teile Kaolin;
c) 25,0 Teile Wirkstoff,
2,5 Teile Isooctylphenoxy-polyoxyäthylenäthanol,
1,7 Teile Champagne-Kreide/Hydroxyäthylcellulose-Gemische (1:1),
8,3 Teile Natriumaluminiumsilikat,
16,5 Teile Kieselgur,
46,0 Teile Kaolin;
d) 10 Teile Wirkstoff,
3 Teile Gemisch der Natriumsalze von gesättigten Fettalkoholsulfaten
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

*Emulgierbare Konzentrate:* Zur Herstellung eines a) 10%igen, b) 25%igen und c) 50%igen emulgierbaren Konzentrates werden folgende Stoffe verwendet:

a) 10,0 Teile Wirkstoff,
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40,0 Teile Dimethylformamid,
43,2 Teile Xylol;
b) 25,0 Teile Wirkstoff,
2,5 Teile epoxydiertes Pflanzenöl,
10,0 Teile eines Alkylarylsulfonat/Fettalkoholpolyglykoläther-Gemisches,
5,0 Teile Dimethylformamid,
57,5 Teile Xylol;
c) 50,0 Teile Wirkstoff,
4,2 Teile Tributylphenol-Polyglykoläther,
5,8 Teile Calcium-Dodecylbenzolsulfonat,
20,0 Teile Cyclohexanon,
20,0 Teile Xylol.

Aus solchen Konzentrationen können durch Verdünnen mit Wasser Emulsionen jeder gewünschten Konzentration hergestellt werden.

*Sprühmittel:* Zur Herstellung eines a) 5%igen und b) 96%igen Sprühmittels werden die folgenden Bestandteile verwendet:

a) 5 Teile Wirkstoff,
1 Teil epoxydiertes Pflanzenöl,
94 Teile Benzin (Siedegrenzen 160-190°C);
b) 95 Teile Wirkstoff,
5 Teile epoxydiertes Pflanzenöl.

*Beispiel 1*

5,4 g 3-Trifluormethyl-4-(1,1,2,2-tetrafluoräthoxy)-anilin werden in 20 ml abs. Äther vorgelegt, und anschliessend werden 3,7 g 2,6-Difluorbenzoylisocyanat, gelöst in 10 ml abs. Äther, bei Raumtemperatur zugetropft. Der ausgefallene Feststoff wird nach 1 Stunde abgesaugt, mit etwas abs. Äther gewaschen und an der Luft getrocknet. Nach Umkristallisation aus Toluol erhält man N-[3-Trifluormethyl-4-(1,1,2,2-tetrafluoräthoxy)-phenyl]-N'-(2,6-difluorbenzoyl)-harnstoff vom Schmelzpunkt 182 bis 185°C.

Analog den vorstehend beschriebenen Arbeitsweisen werden die folgenden Verbindungen der Formel I hergestellt:

| $R_1$ | $R_2$ | $R_3$ | Schmelzpunkt [°C] |
|---|---|---|---|
| $-CF_2-CHF_2$ | F | F | 182-185 |
| $-CF_2-CHF_2$ | Cl | H | 156-157 |
| $-CF_2-CHF_2$ | F | Cl | 146-147 |
| $-CF_2-CHFBr$ | F | F | 187 |
| $-CF_2-CHFBr$ | F | Cl | 137-141 |
| $-CF_2-CHFCl$ | F | F | 189-192 |
| $-CF_2-CHFCl$ | F | Cl | 152-154 |
| $-CF_2-CHF_2$ | Cl | Cl | 167-169,5 |
| $-CHF_2$ | F | F | 167-169 |
| $-CHF_2$ | Cl | Cl | 231-233 |
| $-CHF_2$ | Cl | H | 170-172 |
| $-CF_3$ | Cl | Cl | |
| $-CF_3$ | F | F | |
| $-CF_3$ | Cl | F | |
| $-CF_3$ | F | H | |
| $-CHCl_2$ | F | F | |
| $-CF_2-CHFCl$ | Cl | Cl | 155,5-156,5 |
| $-CF_2-CHFBr$ | Cl | Cl | 146-150 |
| $-CF_2-CHFCl$ | Cl | H | 163-165 |
| $-CF_2-CHFBr$ | Cl | H | 153-155 |
| $-CF_2-CHFBr$ | F | H | 135-136 |
| $-CF_2-CHCl_2$ | Cl | H | 153-154 |
| $-CF_2-CHCl_2$ | F | F | 186-191 |
| $-CF_2-CHF-CF_3$ | F | F | 185-187 |
| $-CF_2-CHF_2$ | F | H | 148-150 |
| $-CF_2-CHFCl$ | F | H | 148-149 |
| $-CF_2-CHFBr$ | F | H | |
| $-CF_2-CHCl_2$ | Cl | Cl | 154-157 |
| $-CF_2-CHCl_2$ | F | Cl | 165-167 |
| $-CF_2-CHCl_2$ | F | H | 148-150 |
| $-CF_2-CHF-CF_3$ | F | Cl | 149-151 |
| $-CF_2-CHF-CF_3$ | Cl | Cl | 154-158 |
| $-CF_2-CHF-CF_3$ | Cl | H | 136-138 |
| $-CF_2-CHF-CF_3$ | F | H | 120-122 |
| $-CF(CF_3)-CHF-CF_3$ | F | F | |
| $-CF(CF_3)-CHF-CF_3$ | F | H | |
| $-CF(CF_3)-CHF-CF_3$ | Cl | Cl | |

*Beispiel 2*

*Wirkung gegen Musca domestica:*

Je 50 frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. nach dem Durchmischen des Substrates lässt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss Beispiel 1 zeigten gute Wirkung im obigen Test.

*Beispiel 3*

*Wirkung gegen Lucilia sericata:*

Zu 9 ml eines Zuchtmediums wurden bei 50°C 1 ml einer 0,5% Aktivsubstanz enthaltenden wässrigen Lösung gegeben. Nun wurden ca. 30 frisch geschlüpfte Lucilia sericata-Larven zum Zuchtmedium gegeben, und nach 48 und 96 Stunden wurde die insektizide Wirkung durch Ermittlung der Abtötungsrate festgestellt.

Verbindungen gemäss Beispiel 1 zeigten in diesem Test gute Wirkung gegen Lucilia sericata.

*Beispiel 4*

*Wirkung gegen Aëdes aegypti:*

Auf die Oberfläche von 150 ml Wasser, das sich in einem Behälter befindet, wurde so viel einer 0,1%igen acetonischen Lösung des Wirkstoffes pipettiert, dass Konzentrationen von je 10, 5 und 1 ppm erhalten wurden. Nach Verdunsten des Acetons wurde der Behälter mit 30-40 2tägigen Aëdes-Larven beschickt. Nach 1, 2 und 5 Tagen wurde die Mortalität geprüft.

Verbindungen gemäss Beispiel 1 zeigen in diesem Test gute Wirkung gegen Aëdes aegypti.

*Beispiel 5*

*Insektizide Wirkung gegen fressende Insekten:*

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven im dritten larvalen Stadium besetzt. Der Versuch wurde bei 28°C und 60% relativer Luftfeuchtigkeit durchgeführt. In Abständen von jeweils 24 Stunden wurden Mortalität sowie Entwicklungs- und Häutungsstörungen der angesetzten Larven bestimmt.

Verbindungen gemäss Beispiel 1 zeigten im obigen Test gute insektizide Wirkung gegen Spodoptera- und Heliothis-Larven.

*Beispiel 6*

*Wirkung gegen Epilachna varivestis:*

Etwa 15-20 cm hohe Phaseolus vulgaris-Pflanzen (Buschbohnen) wurden mit einer wässrigen, den zu prüfenden Wirkstoff enthaltenden Emulsions-Zubereitung besprüht. Nach dem Antrocknen des Sprühbelages wurden pro Pflanze 10 Larven von Epilachna varivestis (Mexikanischer Bohnenkäfer) im 4. larvalen Stadium angesetzt. Über die behandelten Pflanzen wurde ein Plastikzylinder gestülpt, der mit einem Kupfer-Gazedeckel abgedeckt war.

Nach 1 und 2 Tagen wurde die akute Wirkung (% Mortalität) bestimmt. Zur Auswertung hinsichtlich allfälligem Frass-Schaden, (Antifeeding-Effekt),

Entwicklungs- und Häutungsstörungen wurden die Versuchstiere während weiterer 3 Tage beobachtet.

Verbindungen gemäss Beispiel 1 zeigen gute Wirkung in obigem Test.

*Beispiel 7*

*Wirkung gegen Leptinotarsa decemlineata (Larven):*

15 cm hohe in Kulturgefässen befindliche Kartoffelpflanzen, wurden mit einer wässrigen Emulsions-Zubereitung enthaltend den zu prüfenden Wirkstoff in einer Konzentration von 500 ppm, gleichmässig bis zur Tropfnässe mit einer Druckluftspritze besprüht. Nach dem Antrocknen des Spritzbelages auf den Pflanzen, d.h. nach etwa 1,5 Std., wurde über dieselben ein Plastikzylinder gestülpt, und pro Pflanze wurden je 10 Kartoffelkäferlarven des 3. Stadiums angesetzt. Die Zylinder wurden dann mit einem Kupfergaze-Deckel abgeschlossen und in der Dunkelheit bei 28°C und 60% rel. Luftfeuchtigkeit stehen gelassen.

Nach 1 und 2 Std. sowie 1, 2 und 8 Tagen wurden auf Mortalität der Versuchstiere (Rückenlage) und % Frass-Schaden an den Pflanzen geprüft.

Verbindungen gemäss Beispiel 1 zeigten gute Wirkung in obigem Test.

*Beispiel 8*

*Chemosterilisierende Wirkung auf Anthonomus grandis*

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besezten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z.B. «Actamer B 100») desinfiziert und dann in Schalen, die eine geeignete Larvaldiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss Beispiel 1 zeigten gute Wirkung im obigem Test.

**Patentansprüche für den Vertragsstaat:** AT

1. Verbindung der Formel I

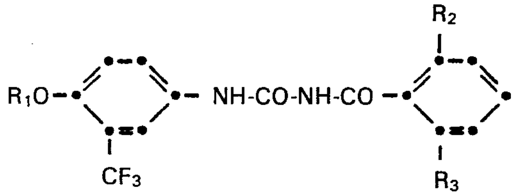

(I)

worin

$R_1$ einen mindestens zweifach mit Fluor, Chlor oder Brom substituierten $C_1$-$C_4$-Alkylrest;

$R_2$ Fluor oder Chlor; und

$R_3$ Wasserstoff, Fluor oder Chlor bedeuten.

2. Verbindung der Formel I gemäss Anspruch 1, dadurch gekennzeichnet, dass $R_2$ und $R_3$ Fluor oder $R_2$ und $R_3$ Chlor oder $R_2$ Fluor oder Chlor und $R_3$ Wasserstoff bedeuten.

3. Verbindung der Formel I gemäss Anspruch 1 oder 2, dadurch gekennzeichnet, dass

$R_1$ die Gruppe $-CF_2-CHX_1X_2$ bedeutet, wobei

$X_1$ für Fluor, Chlor oder Brom und

$X_2$ für Fluor, Chlor, Brom oder Trifluormethyl steht.

4. Verbindung der Formel I gemäss Anspruch 3, dadurch gekennzeichnet, dass $X_1$ Fluor und $X_2$ Fluor, Chlor, Brom oder Trifluormethyl bedeuten.

5. Verbindung der Formel I gemäss Anspruch 4, dadurch gekennzeichnet, dass $X_2$ Chlor oder Brom bedeutet.

6. Verbindung der Formel I gemäss Anspruch 4, dadurch gekennzeichnet, dass $R_1$ die Gruppe $CF_2-CHF-CF_3$ bedeutet.

7. Verbindung gemäss Anspruch 6 der Formel

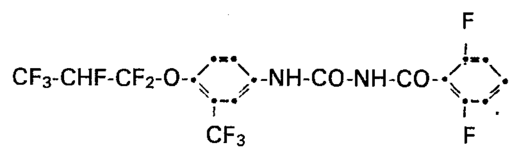

8. Verbindung gemäss Anspruch 6 der Formel

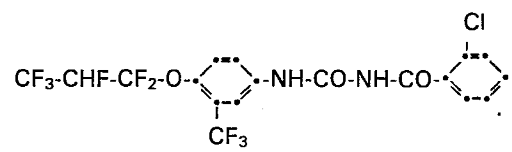

9. Verbindung gemäss Anspruch 6 der Formel

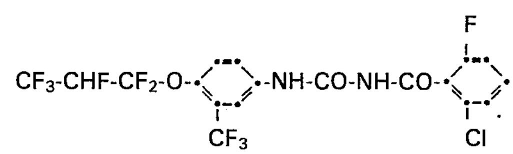

10. Verbindung gemäss Anspruch 4 der Formel

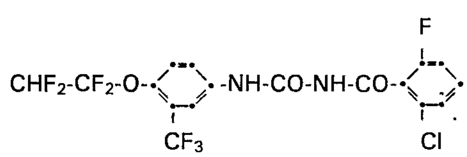

11. Verbindung gemäss Anspruch 5 der Formel

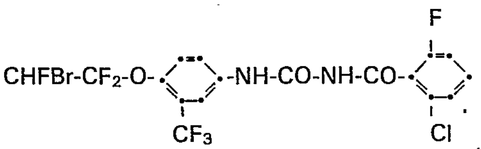

12. Verfahren zur Herstellung einer Verbindung gemäss den Ansprüchen 1 bis 11, dadurch gekennzeichnet, dass man a) eine Verbindung der Formel II

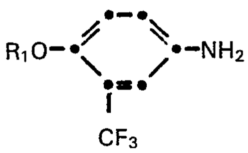
(II)

mit einer Verbindung der Formel III

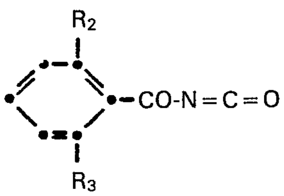
(III)

oder

b) eine Verbindung der Formel (IV)

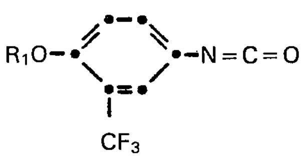
(IV)

mit einer Verbindung der Formel V

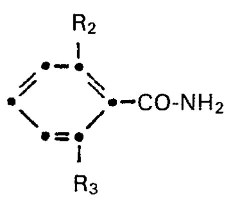
(V)

umsetzt, wobei in den Formeln II bis V die Reste $R_1$, $R_2$ und $R_3$ die in den Ansprüchen 1 bis 6 angegebenen Bedeutungen haben.

13. Schädlingsbekämpfungsmittel, welches als aktive Komponente eine Verbindung gemäss Ansprüchen 1 bis 11 zusammen mit geeigneten Trägern und/oder anderen Zuschlagstoffen enthält.

14. Verwendung einer Verbindung gemäss den Ansprüchen 1 bis 11 zur Bekämpfung von Schädlingen, vorzugsweise von Insekten.

15. Verwendung gemäss Anspruch 14 zur Bekämpfung larvaler Stadien pflanzenschädigender Insekten.

**Patentansprüche für die Vertragsstaaten:**
BE, CH, LI, DE, FR, GB, IT und NL

1. N-Phenyl-N'-Benzoylharnstoffe der Formel I

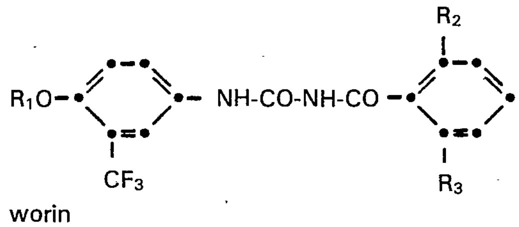
(I)

worin
$R_1$ einen mindestens zweifach mit Fluor, Chlor oder Brom substituierten $C_1$-$C_4$-Alkylrest;
$R_2$ Fluor oder Chlor; und
$R_3$ Wasserstoff, Fluor oder Chlor bedeuten, mit Ausnahme der Verbindung der Formel

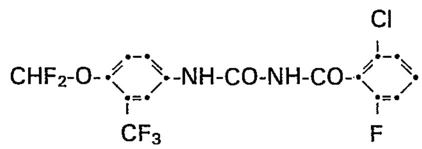

2. Verbindung der Formel I gemäss Anspruch 1, dadurch gekennzeichnet, dass $R_2$ und $R_3$ Fluor oder $R_2$ und $R_3$ Chlor oder $R_2$ Fluor oder Chlor und $R_3$ Wasserstoff bedeuten.

3. Verbindung der Formel I gemäss Anspruch 1 oder 2, dadurch gekennzeichnet, dass
$R_1$ die Gruppe $-CF_2-CHX_1X_2$ bedeutet, wobei
$X_1$ für Fluor, Chlor oder Brom und
$X_2$ für Fluor, Chlor, Brom oder Trifluormethyl steht.

4. Verbindung der Formel I gemäss Anspruch 3, dadurch gekennzeichnet, dass $X_1$ Fluor und $X_2$ Fluor, Chlor, Brom, Brom oder Trifluormethyl bedeuten.

5. Verbindung der Formel I gemäss Anspruch 4, dadurch gekennzeichnet, dass $X_2$ Chlor oder Brom bedeutet.

6. Verbindung der Formel I gemäss Anspruch 4, dadurch gekennzeichnet, dass $R_1$ die Gruppe $-CF_2-CHF-CF_3$ bedeutet.

7. Verbindung gemäss Anspruch 6 der Formel

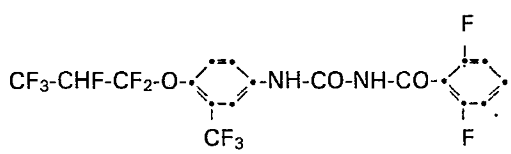

8. Verbindung gemäss Anspruch 6 der Formel

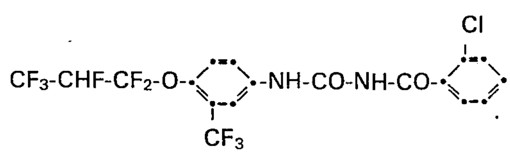

9. Verbindung gemäss Anspruch 6 der Formel

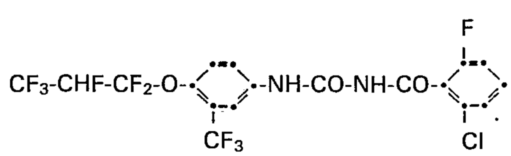

10. Verbindung gemäss Anspruch 4 der Formel

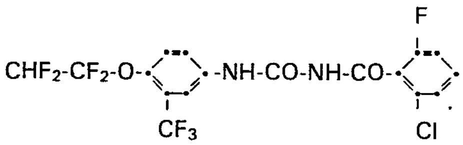

11. Verbindung gemäss Anspruch 5 der Formel

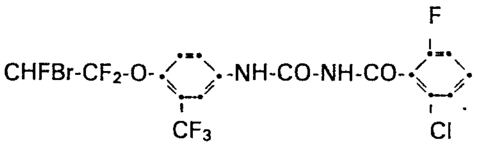

12. Verfahren zur Herstellung einer Verbindung gemäss den Ansprüchen 1 bis 11, dadurch gekennzeichnet, dass man a) eine Verbindung der Formel II

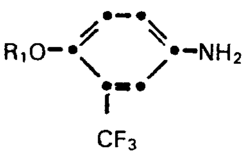 (II)

mit einer Verbindung der Formel III

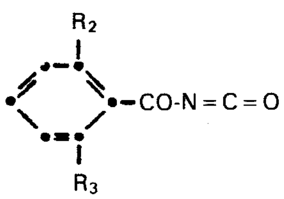 (III)

oder

b) eine Verbindung der Formel (IV)

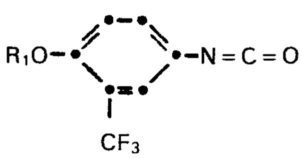 (IV)

mit einer Verbindung der Formel V

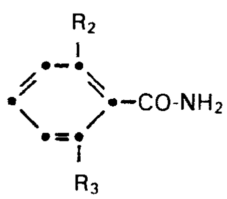 (V)

umsetzt, wobei in den Formeln II bis V die Reste $R_1$, $R_2$ und $R_3$ die in den Ansprüchen 1 bis 6 angegebenen Bedeutungen haben.

13. Schädlingsbekämpfungsmittel, welches als aktive Komponente eine Verbindung gemäss Ansprüchen 1 bis 11 zusammen mit geeigneten Trägern und/oder anderen Zuschlagstoffen enthält.

14. Verwendung einer Verbindung gemäss den Ansprüchen 1 bis 11 zur Bekämpfung von Schädlingen, vorzugsweise von Insekten.

15. Verwendung gemäss Anspruch 14 zur Bekämpfung larvaler Stadien pflanzenschädigender Insekten.

**Claims for the Contracting State:** AT

1. A compound of the formula I

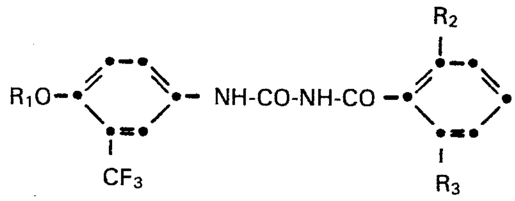 (I)

wherein

$R_1$ is a $C_1$-$C_4$-alkyl group which is substituted at least twice with fluorine, chlorine or bromine,

$R_2$ is fluorine or chlorine, and

$R_3$ is hydrogen, fluorine or chlorine.

2. A compound of the formula I according to claim 1, wherein $R_2$ and $R_3$ are fluorine, or $R_2$ and $R_3$ are chlorine, or $R_2$ is fluorine or chlorine and $R_3$ is hydrogen.

3. A compound of the formula I according to claim 1 or 2, wherein

$R_1$ is the group $-CF_2-CHX_1X_2$, in which

$X_1$ is fluorine, chlorine or bromine, and

$X_2$ is fluorine, chlorine, bromine or trifluoromethyl.

4. A compound of the formula I according to claim 3, wherein $X_1$ is fluorine, and $X_2$ is fluorine, chlorine, bromine or trifluoromethyl.

5. A compound of the formula I according to claim 4, wherein $X_2$ is chlorine or bromine.

6. A compound of the formula I according to claim 4, wherein $R_1$ is the $-CF_2-CHF-CF_3$ group.

7. A compound according to claim 6 of the formula

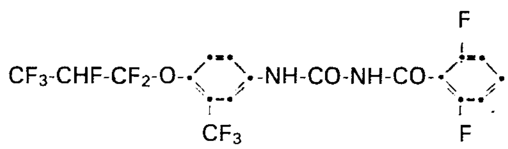

8. A compound according to claim 6 of the formula

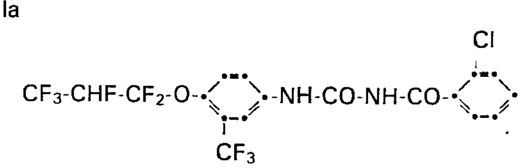

9. A compound according to claim 6 of the formula

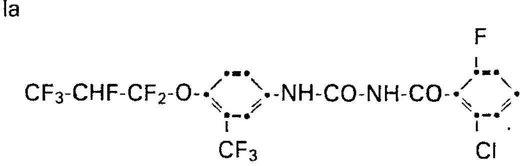

10. A compound according to claim 4 of the formula

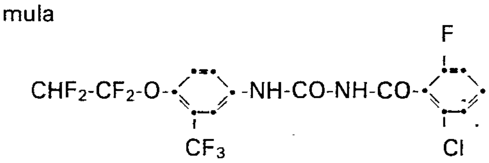

11. A compound according to claim 5 of the formula

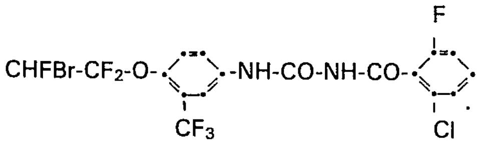

12. A process for producing a compound according to claims 1 to 11 inclusive, which process comprises reacting

a) a compound of the formula II

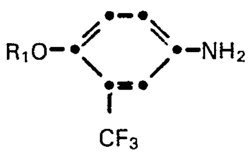

(II)

with a compound of the formula III

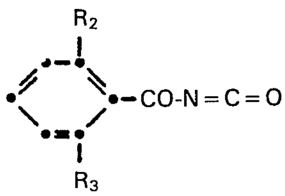

(III)

or

b) a compound of the formula IV

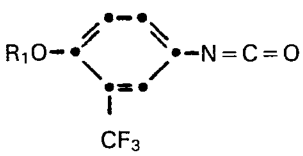

(IV)

with a compound of the formula V

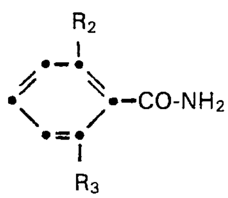

(V)

the radicals $R_1$, $R_2$ and $R_3$ in the formulae II to V having the meanings given in claims 1 to 6.

13. A pesticidal composition containing as active ingredient a compound according to any one of claims 1 to 11 inclusive, together with suitable carriers and/or other additives.

14. Use of a compound according to any one of claims 1 to 11 inclusive for combating pests, particularly insects.

15. Use according to claim 14 for combating larval stages of insects which damage plants.

**Claims for the Contracting States:**
BE, CH, LI, DE, FR, GB, IT and NL

1. An N-phenyl-N'-benzoylurea of the formula I

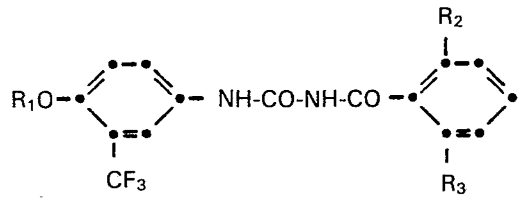

(I)

wherein

$R_1$ is a $C_1$-$C_4$-alkyl group which is substituted at least twice with fluorine, chlorine or bromine,

$R_2$ is fluorine or chlorine, and

$R_3$ is hydrogen, fluorine or chlorine;

with the exception of the compound of the formula

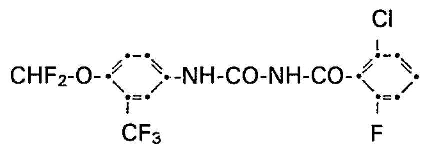

2. A compound of the formula I according to claim 1, wherein $R_2$ and $R_3$ are fluorine, or $R_2$ and $R_3$ are chlorine, or $R_2$ is fluorine or chlorine and $R_3$ is hydrogen.

3. A compound of the formula I according to claim 1 or 2, wherein

$R_1$ is the group $-CF_2-CHX_1X_2$, in which

$X_1$ is fluorine, chlorine or bromine, and

$X_2$ is fluorine, chlorine, bromine or trifluoromethyl.

4. A compound of the formula I according to claim 3, wherein $X_1$ is fluorine, and $X_2$ is fluorine, chlorine, bromine or trifluoromethyl.

5. A compound of the formula I according to claim 4, wherein $X_2$ is chlorine or bromine.

6. A compound of the formula I according to claim 4, wherein $R_1$ is the $-CF_2-CHF-CF_3$ group.

7. A compound according to claim 6 of the formula

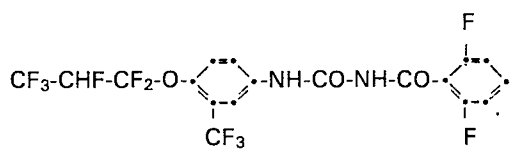

8. A compound according to claim 6 of the formula

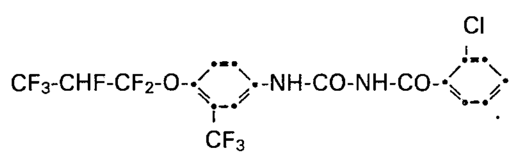

9. A compound according to claim 6 of the formula

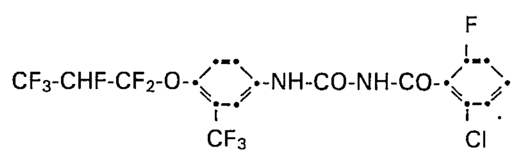

10. A compound according to claim 4 of the formula

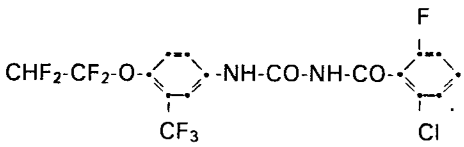

11. A compound according to claim 5 of the formula

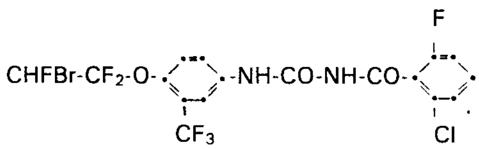

12. A process for producing a compound according to claims 1 to 11 inclusive, which process comprises reacting

a) a compound of the formula II

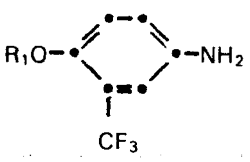

(II)

with a compound of the formula III

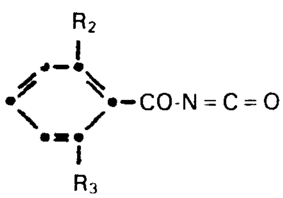

(III)

or

b) a compound of the formula IV

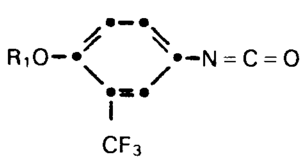

(IV)

with a compound of the formula V

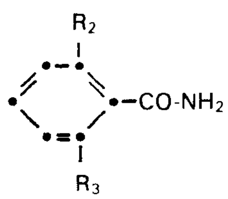

(V)

the radicals $R_1$, $R_2$ and $R_3$ in the formulae II to V having the meanings given in claims 1 to 6.

13. A pesticidal composition containing as active ingredient a compound according to any one of claims 1 to 11 inclusive, together with suitable carriers and/or other additives.

14. Use of a compound according to any one of claims 1 to 11 inclusive for combating pests, particularly insects.

15. Use according to claim 14 for combating larval stages of insects which damage plants.

**Revendications pour l'Etat contractant:** AT

1. Composé de formule I

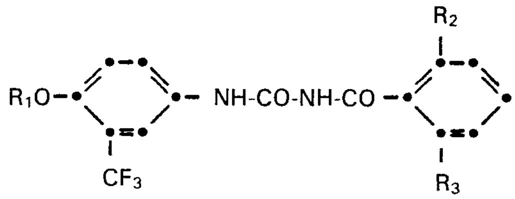

(I)

dans laquelle

$R_1$ représente un groupe alkyle en C1-C4 substitué au moins deux fois par le fluor, le chlore ou le brome,

$R_2$ représente le fluor ou le chlore, et

$R_3$ représente l'hydrogène, le fluor ou le chlore.

2. Composé de formule I selon la revendication 1, caractérisé en ce que $R_2$ et $R_3$ représentent le fluor ou $R_2$ et $R_3$ représentent le chlore ou $R_2$ représente le fluor ou le chlore et $R_3$ l'hydrogène.

3. Composé de formule I selon la revendication 1 ou 2, caractérisé en ce que:

$R_1$ représente le groupe $-CF_2-CHX_1X_2$ dans lequel

$X_1$ représente le fluor, le chlore ou le brome et

$X_2$ représente le fluor, le chlore, le brome ou un groupe trifluorométhyle.

4. Composé de formule I selon la revendication 3, caractérisé en ce que $X_1$ représente le fluor et $X_2$ le fluor, le chlore, le brome ou un groupe trifluorométhyle.

5. Composé de formule I selon la revendication 4, caractérisé en ce que $X_2$ représente le chlore ou le brome.

6. Composé de formule I selon la revendication 4, caractérisé en ce que $R_1$ représente le groupe $CF_2-CHF-CF_3$.

7. Composé selon la revendication 6 de formule

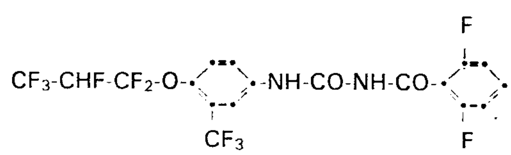

8. Composé selon la revendication 6 de formule

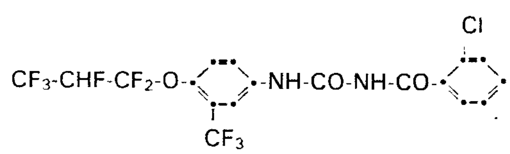

9. Composé selon la revendication 6 de formule

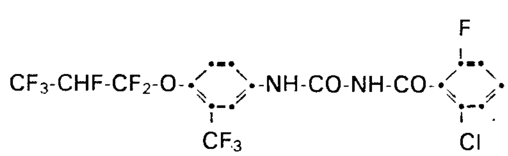

10. Composé selon la revendication 4 de formule

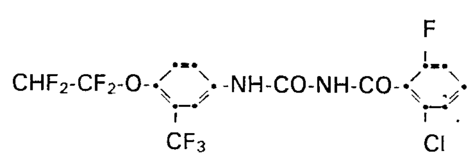

11. Composé selon la revendication 5 de formule

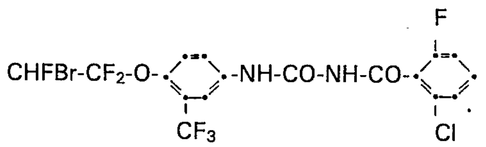

12. Procédé de préparation d'un composé selon les revendications 1 à 11, caractérisé en ce que:
a) on fait réagir un composé de formule II

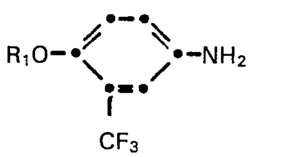 (II)

avec un composé de formule III

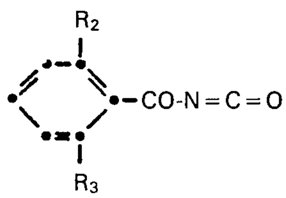 (III)

ou bien
b) on fait réagir un composé de formule IV

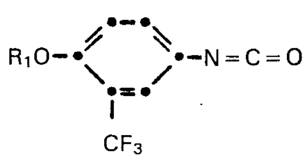 (IV)

avec un composé de formule V

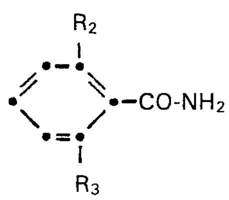 (V)

les symboles $R_1$, $R_2$ et $R_3$ ayant dans les formules II à V les significations indiquées dans les revendications 1 à 6.

13. Produit pesticide contenant en tant que composant actif un composé selon les revendications 1 à 11 avec des véhicules et/ou d'autres additifs appropriés.

14. Utilisation d'un composé selon les revendications 1 à 11 dans la lutte contre les parasites, de préférence les insectes.

15. Utilisation selon la revendication 14, dans la lutte contre les stades larvaires des insectes nuisibles pour les végétaux.

**Revendications pour les Etats contractants:**
BE, CH, LI, DE, FR, GB, IT et NL

1. N-phényl-N'-benzoylurées de formule I

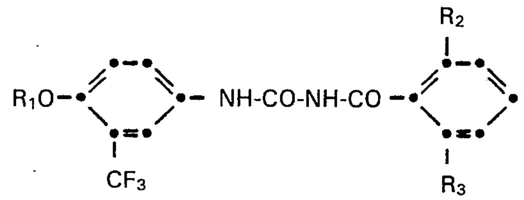 (I)

dans laquelle
$R_1$ représente un groupe alkyle substituéau moins deux fois par le fluor, le chlore ou le brome,
$R_2$ représente le fluor ou le chlore, et
$R_3$ représente l'hydrogène, le fluor ou le chlore, à l'exception du composé de formule

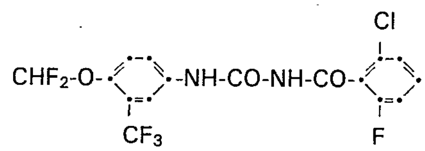

2. Composé de formule I selon la revendication 1, caractérisé en ce que $R_2$ et $R_3$ représentent le fluor ou bien $R_2$ et $R_3$ représentent le chlore ou bien $R_2$ représente le fluor ou le chlore et $R_3$ l'hydrogène.

3. Composé de formule I selon la revendication 1 ou 2, caractérisé en ce que:
$R_1$ représente le groupe $-CF_2-CHX_1X_2$ dans lequel
$X_1$ représente le fluor, le chlore ou le brome et
$X_2$ représente le fluor, le chlore, le brome ou un groupe trifluorométhyle.

4. Composé de formule I selon la revendication 3, caractérisé en ce que $X_1$ représente le fluor et $X_2$ le fluor, le chlore, le brome ou un groupe trifluorométhyle.

5. Composé de formule I selon la revendication 4, caractérisés en ce que $X_2$ représente le chlore ou le brome.

6. Composés de formule I selon la revendication 4, caractérisé en ce que $R_1$ représente le groupe $-CF_2-CHF-CF_3$.

7. Composé selon la revendication 6 de formule

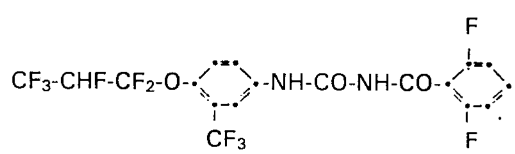

8. Composé selon la revendication 6 de formule

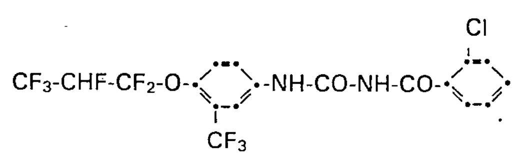

9. Composé selon la revendication 6 de formule

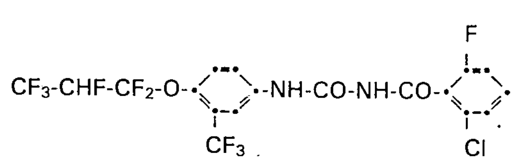

10. Composé selon la revendication 4 de formule

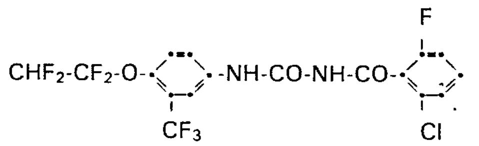

11. Composé selon la revendication 5 de formule

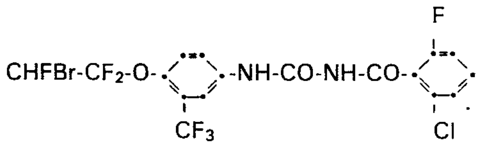

12. Procédé de préparation d'un composé selon les revendications 1 à 11, caractérisé en ce que:
a) on fait réagir un composé de formule II

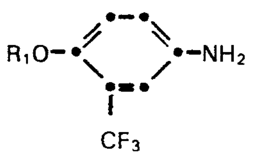 (II)

avec un composé de formule III

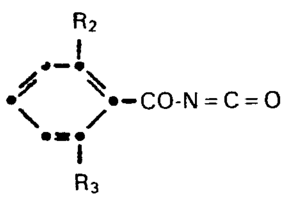 (III)

ou bien
b) on fait réagir un composé de formule IV

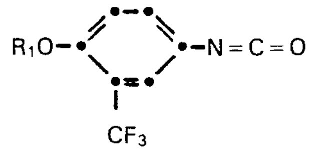 (IV)

avec un composé de formule V

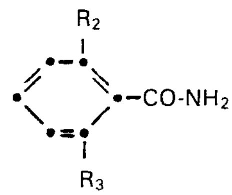 (V)

les symboles $R_1$, $R_2$ et $R_3$ ayant dans les formules II à V les significations indiquées dans les revendications 1 à 6.

13. Produit pesticide contenant en tant que composant actif un composé selon les revendications 1 à 11 avec des véhicules et/ou d'autres additifs appropriés.

14. Utilisation d'un composé selon les revendications 1 à 11 dans la lutte contre les parasites, de préférence les insectes.

15. Utilisation selon la revendication 14, dans la lutte contre les stades larvaires des insectes nuisibles pour les végétaux.